# EUROPÄISCHE PATENTANMELDUNG

(11) **EP 2 182 423 A2**
(43) Veröffentlichungstag der Anmeldung: **05.05.2010**
(21) Anmeldenummer: 09174119.9
(22) Anmeldetag: 27.10.2009
(51) Int. Cl.: G06F 3/01, G06F 3/033

(54) **Schreibvorrichtung**

(30) Priorität: 28.10.2008 DE 102008037487
(71) Anmelder: Karlsruher Institut für Technologie, 76131 Karlsruhe (DE)
(72) Erfinder: Blank, Rainer, 75433, Maulbronn (DE); Hoffmann, Jan, 76744, Wörth (DE); Prokop, Jürgen, 76829, Landau (DE); Ritzhaupt-Kleissl, Hans-Joachim, 69190, Walldorf (DE)
(74) Vertreter: Fitzner, Uwe

(57) **Zusammenfassung**

Schreibvorrichtung umfassend Sensoren zur Erfassung biometrischer Daten und Vorrichtungen zur Abgabe von haptischer Rückmeldung, sowie deren Verwendung

## Beschreibung

Die vorliegende Anmeldung nimmt die Priorität der DE 10 2008 037 487.3 in Anspruch.
Das Prioritätsdokument ist durch Verweis vollumfänglich in die vorliegende Offenbarung einbezogen (= incorporated by reference in its entirety).

Alle in der vorliegenden Anmeldung zitierten Dokumente sind durch Verweis vollumfänglich in die vorliegende Offenbarung einbezogen (= incorporated by reference in their entirety).

Die vorliegende Erfindung betrifft eine Schreibvorrichtung, deren Verwendung zur Kontrolle der Schreibhaltung sowie deren Verwendung zur Diagnose und zur Therapie neuromotorischer Störungen, insbesondere graphomotorischer Störungen.

Neuromotorische Krankheiten werden hauptsächlich durch altersbedingte Krankheiten, durch Unfälle oder durch physischen oder mentalen Stress hervorgerufen. Auch Alkohol- oder Drogenprobleme oder Behandlung mit bestimmten Medikamenten können entsprechende Probleme hervorrufen.

Unter dem Begriff "Graphomotorik" werden allgemein alle Prozesse eingeordnet, die zu einer Produktion von grafischen Zeichen mittels der Hand und einem geeigneten Schreibgerät auf einem Untergrund führen. Dazu zählen vor allem die Tonusregulation (Muskelspannung) und Kraftdosierung bei der Stifthaltung sowie die korrekte Körper- und Sitzhaltung. Neuromotorische Störungen führen in aller Regel auch zu graphomotorischen Störungen, da der Schreibvorgang ein sehr komplexer Vorgang ist, der selbständiges Koordinieren von Händen, Tastsinn und visueller Wahrnehmung voraussetzt. Das Schreiben ist eine der schwierigsten motorischen Fähigkeiten des Menschen.

Kinder im Grund- und Vorschulalter haben häufig Schwierigkeiten beim Umgang mit Schreibstiften und dem Erlernen des Schreibvorgangs. Die Fehlerquellen sind vielfältig und reichen vom Verkrampfen der Hand über eine falsche Führung des Schreibgeräts bis zur Anwendung zu hoher axialer und radialer Drucke.

Derzeit können solche Störungen und Schwierigkeiten kaum mit medizinischen Geräten diagnostiziert werden, sondern es bedarf in hohem Umfang der Erfahrung des Therapeuten bei der optischen Beobachtung des Schreibvorgangs des Patienten. Zur Unterstützung der Therapie werden Übungsbögen eingesetzt, auf denen der Patient bestimmte Formen nachzeichnen muss, die dann ausgewertet werden. In jedem Fall erfolgt eine Fehlerkorrektur mündlich durch den Therapeuten und verlangt vom Patienten eine Transferleistung dergestalt, dass die Empfehlungen des Therapeuten in einem erneuten Therapieschritt umgesetzt werden. Dazu sind insbesondere Kinder im Vorschulalter, also in dem Alter, in dem der Schreibvorgang erlernt wird, häufig noch nicht in der Lage.

In neueren Therapieformen werden unter anderem Federn eingesetzt um direkte Rückmeldungen an die Patienten zu liefern. Die für die Therapie essentiellen Beschleunigungen, die bei der Stiftbewegung entstehen können, werden jedoch dabei und auch bei den bestehenden anderen Systemen nicht detektiert. Auch die Art der Stiftspitze kann in diesen Fällen nicht an das Alter und die individuelle Entwicklungsstufe der Patienten angepasst werden. Für die jungen Patienten ist es dabei anfangs erstrebenswert Farbstifte einzusetzen, jedoch im Laufe der Untersuchungen die Stiftspitze bis hin zu Füllerspitzen zu ersetzen. Nachteilig ist hierbei oftmals auch die notwendige Kabelführung oder die Möglichkeit die Art der Übertragung an das Umfeld anzupassen. Die Rückmeldung an den Patienten, welcher Wert angepasst werden muss, um das gewünschte Ergebnis zu erzielen bleibt in diesem Fall auch ungeachtet.

Aus der DE 10 2005 031 432 ist ein fluidischer Stift zur Erfassung neuromotorischer Daten bekannt. Dieser weist eine fluidgefüllte Kammer auf, die vorzugsweise einen ersten ring- oder spiralfömigen Abschnitt und einen zweiten kanalförmigen Abschnitt aufweist. Dabei entspricht der Flüssigkeitsdruck innerhalb der Fluidkammer einer bei einer handgeführten Bewegung auftretenden Kraft. Über einen Drucksensor wird der Flüssigkeitsdruck erfasst und in ein elektrisches Signal für eine Datenverarbeitungseinheit umgewandelt. Durch Anwendung mehrere Drucksensoren können radiale Kräfte und Beschleunigungswerte ermittelt werden, doch ist eine saubere Trennung dieser beiden Kräfte nur über einen Rechenalgorithmus möglich, da der Flüssigkeitsdruck in der Kammer von beiden Kraftarten bestimmt wird. Über den Drucksensor am Ende des kanalförmigen Endes der Fluidkammer können auch axiale Kräfte bestimmt werden, allerdings wiederum nicht eindeutig getrennt von radialen oder Beschleunigungskräften. Die gemessenen Daten werden an eine Datenverabeitungseinheit übermittelt, die in dem Stift integriert oder als externe Komponente vorgesehen sein kann. In dieser Datenverarbeitungseinheit werden die ermittelten Daten gespeichert und nach Übertragung auf einen Computer mit Hilfe einer entsprechenden Software durch den Therapeuten ausgewertet. Anschließend werden mit der betreffenden Person, von der die Daten stammen, im Gespräch mögliche Störungen diskutiert und ggf. Handlungsempfehlungen zur Therapie gegeben. Eine unmittelbare Rückmeldung an den Patienten während der Erfassung der Daten ist nicht möglich.

Aus der DE 10 2005 017 936 ist ein fluidisches Peripherie-Eingabegerät für eine elektronische Datenverarbeitungseinrichtung bekannt, bei dem mittels einer Fluidkammer ein axialer Druck gemessen wird, welcher der Kraft entspricht, mit der das Eingabegerät auf der Unterlage aufgesetzt wird. Vorteil dieses Systems als PC-Peripherieeingabegerät ist, dass die Eingabe der Daten ergonomisch einfach und räumlich autonom erfolgen kann. Da weder radiale Drucke noch Beschleunigungswerte ermittelt werden (diese spielen für die vorgesehene Anwendung keine Rolle) ist dieses Gerät zur Diagnose oder Therapie neuromotorischer Störungen nicht geeignet. Ein irgendwie geartete Rückmeldung während des Erfassungsvorgangs erfolgt nicht, da diese für den vorgesehenen Anwendungszweck nicht erforderlich ist.

Aus DE 20 2005 019 142 ist ein Schreibgerät mit optischer Kontrolle der Aufdruckkraft bekannt, in welchem ein federungsgelagertes Schreibmittel nach Übersteigen einer definierten Aufdruckkraft einen Stromschlusskontakt zu einer Energiequelle und einem Leuchtmittel herstellt, wodurch das Leuchtmittel ein optisches Signal aussendet. Radiale Kräfte oder Beschleunigungskräfte werden nicht gemessen, was die Eignung des Stifts für eine erfolgversprechende Therapie neuromotorischer Störungen ungeeignet macht. Die rein optische Rückmeldung hat zudem den Nachteil, dass, insbesondere bei der Therapie von Kindern die Aufmerksamkeit des Patienten nur noch auf das optische Signal ausgerichtet ist und damit zu einer für den Patienten unnatürlichen Schreibhaltung führen kann.

Schreibvorrichtungen oder Schreibsysteme sind beispielsweise aus der DE 102 41 328, der DE 10 2005 031 432, der US 5,247,137 oder den deutschen Gebrauchsmustern DE 20 2005 017 936 und DE 20 2007 002 778 bekannt. Alle diese bekannten Schreibvorrichtungen sind zur Erfassung biometrischer Daten geeignet, weisen aber keine Möglichkeit einer direkten Rückmeldung auf.

Aufgabe der vorliegenden Erfindung war es, eine Vorrichtung zur Verfügung zu stellen, mit welcher die Schreibhaltung kontrolliert werden kann, sowie eine Vorrichtung zur Verfügung zu stellen, welche geeignet ist, bei der Diagnose und Therapie neuromotorischer Störungen, insbesondere von graphomotorischen Störungen bei Kindern im Vorschulalter verwendet zu werden und die die vorstehend geschilderten Nachteile nicht aufweisen.

Diese Aufgabe wird erfindungsgemäß durch Vorrichtungen und Verwendungen gemäß der Ansprüche, sowie gemäß der nachfolgenden Beschreibung und Figuren gelöst.

Bevorzugte Ausführungsformen der Erfindung sind den Unteransprüchen und der nachfolgenden detaillierten Beschreibung zu entnehmen.

Die Begriffe "Kraftsensor" und "Drucksensor" werden im Rahmen der vorliegenden Erfindung synonym verwendet.

Mit der erfindungsgemäßen Vorrichtung werden die Krafteinwirkungen und Beschleunigungen aus der handgeführten Bewegung des Nutzers kontinuierlich erfasst. Diese erfassten Daten werden in einer Datenverarbeitung kontinuierlich auf eine Abweichung mit vorher gespeicherten Referenzdaten geprüft, wobei im Falle einer Abweichung von den vorher festgelegten Referenzdaten unmittelbar eine Meldung generiert wird, die zu einer haptisch spürbaren und optischen, akustischen oder optisch-akustischen Rückmeldung für den Nutzer führt, solange die Abweichung von den Referenzdaten anhält.

Die erfindungsgemäße Schreibvorrichtung eignet sich damit hervorragend zur Kontrolle der Schreibhaltung bzw. zur Kontrolle der Griffhaltung des jeweiligen Nutzers.
Durch diese Kontrolle kann in einer Variante der vorliegenden Erfindung die erfindungsgemäß Schreibvorrichtung im Rahmen eines Diagnose- bzw. Therapie-Verfahrens eingesetzt bzw. verwendet werden.
In einer anderen Variante der vorliegenden Erfindung kann die erfindungsgemäße Schreibvorrichtung auch für nicht-therapeutische Zwecke eingesetzt werden.

Es ist bei der erfindungsgemäßen Vorrichtung möglich, je nach Nutzer geeignete Schreibstiftspitzen und Übertragungseinheiten auszuwählen bzw. an die jeweiligen Bedürfnisse und Erfordernisse des Nutzers anzupassen.

Sofern die erfindungsgemäße Schreibvorrichtung in einem Verfahren zur Diagnose und Therapie verwendet wird, macht sie es dem Therapeuten möglich je nach Patient eine geeignete Stiftspitze und Übertragungseinheit auszuwählen und als Therapieeinheit zur Verfügung zu stellen.

Die erfindungsgemäße Schreibvorrichtung macht es möglich, Therapieschritte bei der Behandlung neuromotorischer Störungen ohne direkte Mitwirkung oder Anwesenheit eines Therapeuten, beispielsweise in der vertrauten häuslichen Umgebung des Patienten durchzuführen. Bei der Durchführung von Übungen zur Verbesserung seiner Schreibfähigkeit erhält der Nutzer eine unmittelbare haptisch spürbare und optische oder akustische Rückmeldung über Fehler beim Aufdrücken des Stifts, bei der Stifthaltung oder bei ruckartiger Bewegung der Schreibvorrichtung und kann, indem ihm angezeigt wird, ob der Druck, die Stifthaltung oder die Stiftführung den Fehler verursacht haben, eine unmittelbare Korrektur des Fehlers vornehmen, indem er Druck, Stifthaltung oder Stiftführung dergestalt variiert, dass die haptisch spürbare oder akustische Rückmeldung verschwindet, was der Fall ist, sobald die kontinuierlich erfassten Daten wieder im Referenzbereich liegen, der vorher festgelegt und in der Datenverarbeitungseinheit gespeichert wurde. Dabei kann der Therapeut in Abhängigkeit von der Schwere der neuromotorischen Störung die Referenzwerte in mehreren Schritten an die Idealwerte heranführen, um insbesondere bei Kindern, diesen bei Fortschritten das wichtige Erfolgserlebnis zu verschaffen, wenn eine Verbesserung zu erkennen ist, aber dennoch weitere Anstrengungen erforderlich sind. So kann schrittweise mit den kleinen Patienten auf die Erreichung der Idealwerte hingearbeitet werden.

Vorzugsweise handelt es sich bei der haptisch spürbaren Rückmeldung an den Nutzer um eine Vibration mit optischem Signal, da eine derartige Rückmeldung den Vorteil bietet, eine Fortsetzung der fehlerhaften Schreibbewegung wirksam zu verhindern und andererseits der Nutzer nicht gezwungen ist, zum Erkennen der Rückmeldung seine Aufmerksamkeit vom eigentlichen Schreibvorgang abzuwenden, wie dies beispielsweise bei rein optischen oder numerischen Rückmeldungen auf einer Skala der Fall ist. Durch die zusätzliche optische Signalgebung wird der Nutzer auch darauf hingewiesen, welcher der notwendigen Werte sich nicht im Referenzbereich befindet. Der Nutzer kann seine ganze Konzentration auf die Schreibbewegung und deren Verbesserung richten, ohne Ablenkungen zu unterliegen. Durch Steuerung der Intensität der Vibration, erhält der Nutzer sogar, ohne seine Konzentration von der Schreibbewegung abwenden zu müssen, einen Hinweis auf den Grad der Abweichung vom Ideal- oder Sollzustand.

Vibrationen können mit kleinen Elektromotoren erzeugt werden, die über eine Excenter-Masse Schwingungen an die Schreibvorrichtung weitergeben. Die optische Signalgebung erfolgt über mindestens drei Lichtsignale, die jeweils für die radialen Kräfte, axiale Kraft und die Beschleunigungswerte stehen. Bevorzugt wird diese so gestaltet, dass dem Nutzer durch verschiedenfarbige optische Rückmeldungen beispielsweise wie bei einer Verkehrsampel durch ein grünes Signal gemeldet wird, dass er sich im richtigen Bereich befindet. Mit einer gelben oder orangefarbenen Rückmeldung kann dann signalisiert werden, dass der Grenzwert der vorgesehenen Bereiche erreicht ist und schließlich kann eine Rückmeldung in Rot signalisieren, dass Korrekturbedarf besteht. Auf diese Weise der optischen Rückmeldung mit verschiedenfarbigen Anzeigen kann dem Nutzer während des Schreibvorgangs eine kontinuierliche Rückmeldung gegeben werden, wenn er die Schreibhaltung in unerwünschter Weise verändert. Es versteht sich, dass natürlich auch beliebige andere Farben für die optische Rückmeldung eingesetzt werden können.

Auch geeignete Systeme zur Erzeugung akustischer Rückmeldungen sind dem Fachmann bekannt und in der Literatur beschrieben, so dass sich hier weitere Einzelheiten erübrigen. Auch hier wird durch die Ausgestaltung des Signals deutlich gemacht, welcher der Werte überschritten wurde. Als Anreiz kann bei Kindern als Benutzern oder Patienten die Elektronik derart programmiert werden, dass am Ende einer festgelegten Übungsdauer oder bei Unterschreitung einer vorher festgelegten Fehlerquote eine Melodie ertönt. So werden dem Kind Erfolgserlebnisse vermittelt, die für die Motivation oder die Therapie und deren Akzeptanz durch das Kind vorteilhaft sind.

Im Rahmen der vorliegenden Erfindung können auch mehrere Arten der Rückmeldung kombiniert werden, solange mindestens eine akustische oder haptisch spürbare Rückmeldung angewandt wird. So kann z.B. eine optische Rückmeldung vorgesehen werden, um dem Nutzer anzuzeigen, wenn er sich innerhalb der vorgesehenen Bereiche bewegt; beim Verlassen der vordefinierten Bereiche erfolgt dann eine haptisch spürbare oder akustische Rückmeldung zusätzlich.

In einer Variante der vorliegenden Erfindung erfolgt die Prüfung der erfassten Daten auf Abweichung von vorher festgelegten Referenzdaten in einer in die Schreibvorrichtung integrierten Datenverarbeitungseinheit, vorzugsweise einem Mikroprozessor. Bevorzugt verwendbar sind sogenannte Single-Chip-Controller, die im Unterschied zu den gängigen PC-Prozessoren keine weitere Hardware benötigen, um die implementierten Funktionen zu erfüllen. Nur beispielhaft seien hier die Atmega-Prozessoren nach der AVR-Architektur erwähnt, die es in verschiedenen Ausführungen gibt, die sich hinsichtlich Prozessortakt, Anzahl der Ausgänge und Bauform unterscheiden. Diese Chips können bei maschinell gefertigten Leiterplatinen ohne Gehäuse verbaut werden, was eine sehr kleine Bauweise ermöglicht, wie sie für den Einbau in eine Schreibvorrichtung, deren äußere Form einem üblichen Stift möglichst nahe kommen soll, erforderlich ist. Bei dieser Ausführungsform werden daher keinerlei weitere Geräte mehr benötigt, um eine kontinuierliche Analyse der Schreibbewegung durchzuführen. Entsprechende Prozessoren sind dem Fachmann bekannt und in der Literatur beschrieben.

In einer alternativen Variante kann der Vergleich der erfassten Daten auch in einer externen Datenverarbeitungseinheit erfolgen, beispielsweise mit einem handelsüblichen Personal Computer oder einem PDA (Personal Digital Assistant), wie sie dem Fachmann bekannt sind. Bei dieser Ausführungsform des erfindungsgemäßen Verfahrens werden die Daten von der Schreibvorrichtung zur externen Datenverarbeitungseinheit übertragen. Dazu kann wiederum ein Mikroprozessor in der Schreibvorrichtung verwendet werden. Die Übertragung an die externe Datenverarbeitungseinheit kann dabei über eine kabelgebundene Verbindung erfolgen. Während dies technisch die am einfachsten zu realisierende und in den meisten Fällen auch kostengünstigste Variante ist, hat sie den Nachteil, dass die Kabelverbindung zwischen Schreibvorrichtung und Datenverarbeitungseinheit vom Nutzer bei der Durchführung der Schreibbewegungen als störend empfunden wird und ein natürliches Schreibgefühl nicht entsteht. Dies kann zu Artefakten führen, da die gemessenen Ergebnisse unter Umständen nicht mehr denen entsprechen, die bei einer natürlichen Schreibbewegung ohne störendes Kabel erhalten werden.

In den meisten Fällen ist es daher bevorzugt, bei Verwendung der erfindungsgemäßen Schreibvorrichtung mit einer externen Datenverarbeitungseinheit die Daten mit Hilfe eines drahtlosen Verfahrens zu übertragen.

Als Beispiel wäre hier zunächst die Infrarot-Funkübertragung zu nennen, deren Prinzip dem Fachmann bekannt ist. Die Hardware-Implementierung eines Infrarot-Übertragungssystems ist einfach, die Softwareimplementierung jedoch relativ komplex. Die Sendediode strahlt in der Regel mit einem Öffnungswinkel von etwa 30° und einer Wellenlänge im Bereich von 850 und 900 nm ab. Nachteilig bei dieser Technik ist die relativ geringe Reichweite (in der Regel nicht mehr als ein Meter bei der erforderlichen Miniaturisierung) und die erforderliche Sichtverbindung zwischen Sender und Empfänger.

Als weitere Möglichkeit ist hier der dem Fachmann bekannte und in der Literatur beschriebene Bluetooth-Übertragungsstandard zu nennen, der speziell zur drahtlosen Kurzstreckenkommunikation zwischen mehreren Geräten entwickelt wurde und sich mittlerweile als Quasi-Standard im Bereich der Mobiltelefone durchgesetzt hat. Bluetooth verwendet das Industrial Scientific Medicine (ISM)-Funkband bei 2,4 GHz. Die Verwendung in Krankenhäusern und Arztpraxen ist erlaubt und eine Störung anderer medizinischer Geräte ausgeschlossen.

Geeignete Empfänger sind in vielen PC und insbesondere in sogenannten Handhelds oder Personal Digital Assistants (PDA) bereits standardmäßig eingebaut.

Prinzipiell ist auch eine Übertragung über den Standard möglich, der als Wireless LAN (IEEE 802.11) bekannt ist und der sich als Standard für den Aufbau drahtloser Netzwerke mehrerer Personal Computer bewährt hat. Praktisch jeder PC ist heute mit einer entsprechenden Empfangseinheit ausgerüstet oder einfach nachrüstbar. Wireless LAN ermöglicht eine Übertragung über größere Entfernungen als Bluetooth oder Infrarot, hat aber den erheblichen Nachteil des deutlich höheren Strombedarfs, was zu nur kurzen Laufzeiten führt, wenn der Stift nicht eine entsprechende Stromversorgung hat. Zudem ist die Verwendung von Wireless LAN in Kliniken und Arztpraxen nicht allgemein erlaubt.

Ohne hier im Detail näher darauf einzugehen, seien noch zwei weitere telemetrische Übertragungsverfahren erwähnt, die unter den Bezeichnungen simpliciTI-Protokoll bzw. ZigBee dem Fachmann bekannt und in der Literatur beschrieben sind. Beide arbeiten im gleichen Frequenzband und haben nur einen niedrigen Strombedarf. Die Verbreitung dieser Techniken ist jedoch geringer als beispielsweise Bluetooth.

Als biometrische Daten werden von der erfindungsgemäßen Schreibvorrichtung vorzugsweise axiale, radiale und Beschleunigungskräfte gemessen.
Der axiale Druck, der im Rahmen der vorliegenden Erfindung gemessen wird, entspricht dem Anpressdruck bzw. der Anpresskraft der Schreibvorrichtung auf die Schreibunterlage.
Der radiale Druck, der im Rahmen der vorliegenden Erfindung gemessen wird, entspricht dem Griffdruck, der durch den Benutzer auf die Schreibvorrichtung ausgeübt wird.
Axiale Drücke entstehen also beim Aufsetzen eines Schreibgeräts auf die Schreibfläche und ermöglichen eine Aussage darüber, ob der Patient den Stift mit zu hohem Druck auf dem Papier führt, was im Extremfall zum Zerreißen des Papiers oder dem Abbrechen der Stiftspitze führen kann.
Radiale Drücke entstehen also beim Erfassen des Schreibgeräts, wozu in der Regel Daumen, Zeigefinger und Mittelfinger verwendet werden. Greift der Patient mit zu hohem Druck das Schreibgerät treten schnell schmerzhafte Ermüdungserscheinungen auf und es kann bis zu physischen Druckstellen an den Fingern, die das Schreibgerät halten, kommen.

Die Erfassung der axialen und/oder radialen Drücke erfolgt mit Hilfe von Drucksensoren, wie sie dem Fachmann an sich bekannt und in der Literatur beschrieben sind.

Grundlage der meisten Drucksensoreffekte ist die elastische Verformung eines Festkörpers.
Die sogenannten Dehnungsmessstreifen beruhen auf der Widerstandsänderung durch Verformung eines elektrischen Leiters. Der daraus resultierende Spannungsabfall wird im Messgerät detektiert.
Bei piezoresistiven Drucksensoren handelt es sich im Prinzip auch um Dehnungsmessstreifen, die auf eine bewegliche Membran oder eine Messzelle im Innern einer Membran aufgebracht werden und darüber indirekt den Druck messen.

Beide vorgenannten Methoden weisen eine hohe Temperaturabhängigkeit auf, was in jedem Fall eine Eichung und genaue Temperaturkontrolle erforderlich macht.
Piezoelektrische Drucksensoren beruhen darauf, dass bei Ausüben von Druck auf Quarzkristalle durch Veränderungen in der Kristallstruktur an der Oberfläche eine elektrische Ladung entsteht. Diese Technik kann sehr gut miniaturisiert werden, erfordert jedoch einen Messverstärker, da die Nutzsignale sehr klein sind.
Kraftsensoren mit elektromagnetischer Kompensation beruhen auf der Kompensation einer Kraft, die auf einen Eisenkern oder Anker aufgebracht wird, durch eine elektrische Schaltung, wobei die zur Kompensation der aufgebrachten Kraft erforderliche Spannung direkt proportional der aufgebrachten Kraft ist. Der Aufbau ist jedoch komplex und aufwändig und auch die erforderliche Miniaturisierung erfordert Aufwand.
Kapazitive Kraftsensoren beruhen auf dem Prinzip eines elektrischen Kondensators, der mit der Änderung des Plattenabstandes (durch eine einwirkende Kraft auf eine der Platten) seine Kapazität ändert. Es ist darauf zu achten, dass es nicht bei den gemessenen Drucken zu einer Berührung der beiden Kondensatorplatten kommt, da dies zu einem Ausfall des Sensors durch Kurzschluss führt.
In vielen Fällen haben sich als Drucksensoren für Anwendungen der hier in Rede stehenden Art jedoch sogenannte Foliendrucksensoren bewährt, die demgemäß auch im erfindungsgemäßen Verfahren bevorzugt eingesetzt werden. Das Prinzip beruht auf der Widerstandsänderung einer Halbleiterfolie bei Anwendung von äußerem Druck.
Drucksensoren der vorstehend kurz geschilderten Art sind in vielfacher Ausfertigung in der Literatur beschrieben und im Handel erhältlich.

Für die Messung von Beschleunigungskräften stehen auch verschiedene Arten von Sensoren zur Verfügung. Nur beispielhaft seien hier piezoelektrische Sensoren erwähnt, die in der Mikrotechnik breite Anwendung finden. Beim Wirken einer Beschleunigung auf die seismische Masse eines solchen Sensors ändern die Piezo-Widerstände ihre Leitfähigkeit und durch den Spannungsabfall kann auf die einwirkenden Beschleunigungskräfte geschlossen werden.
Bei den sogenannten kapazitiven Beschleunigungssensoren beruht die Messung auf der Lageänderung der seismischen Masse, die Kapazitätsänderungen zur Folge hat.
Schließlich seien noch kraftkompensierte Beschleunigungssensoren erwähnt, bei denen die äußere Kraft durch eine entsprechende Schaltung kompensiert wird.
Auch geeignete Beschleunigungssensoren der vorstehend beschriebenen Art sind in vielfacher Ausfertigung kommerziell erhältlich und in der Literatur beschrieben.

Vorzugsweise werden die Beschleunigungskräfte in mindesten zwei Raumachsen, bevorzugt in allen drei Achsen gemessen und erfasst, um eine möglichst wirksame Therapie durchführen zu können.

Um dem Therapeuten oder Korrektor, der bei der Durchführung der Schreibübungen nicht anwesend sein muss und im Regelfall auch nicht anwesend sein wird, eine Auswertung zu ermöglichen, können die erfassten Daten in einer bevorzugten Ausführungsform der vorliegenden Erfindung in einem Datenspeicher abgelegt werden, der vom Therapeuten oder Korrektor ausgelesen werden kann.

Bevorzugt werden erfindungsgemäße Schreibvorrichtungen eingesetzt, die modular aufgebaut sind, d.h. aus mehreren wechselbaren Modulen bestehen, und aus einem Schreibstiftgehäuse zur Durchführung handgeführter Bewegungen, mindestens zwei Drucksensoren zur Messung von axialen und radialen Drücken und einen Beschleunigungssensor zur Messung von Beschleunigungskräften, eine Datenverarbeitungseinheit und eine Einrichtung zur Erzeugung und Abgabe eines haptisch spürbaren oder akustischen Signals sowie eine Energiequelle aufweisen. Gegebenenfalls kann noch ein Datenspeicher, z.B. ein mobiler Datenspeicher zur Speicherung der Daten zur späteren Auswertung an einem Computer oder PDA vorhanden sein.

Eine alternative erfindungsgemäße Schreibvorrichtung weist anstelle der integrierten Datenverarbeitungseinheit eine Einrichtung zur Übertragung der erfassten Daten an eine externe Datenverarbeitungseinheit auf, ist im übrigen aber wie vorstehend aufgebaut.

Gemäß einer besonders bevorzugten Ausführungsform ist die erfindungsgemäße Schreibvorrichtung modular aufgebaut, d.h. besteht aus mehreren auswechselbaren Modulen. So hat es sich bewährt, die Stiftspitze der erfindungsgemäßen Schreibvorrichtung mit den verwendeten Sensorsystemen als ein Modul zu gestalten. Darin enthalten ist ein Drucksensor zur Erfassung der axialen Drücke, die Drucksensoren zur Messung der radialen Drücke, die um die äußere Hülle der Stiftspitze angeordnet sind und die Beschleunigungssensoren. Dies ermöglicht den einfachen Austausch der Spitzen um verschiedene Schreibmienen verwenden zu können. Der Austausch der Schreibmine in einem System, in dem der axiale Drucksensor nicht mit der Mine gekoppelt ist, birgt das Risiko von fehlerhaften Messungen, wenn die neu eingesetzte Mine keinen richtigen Anschluss an den Drucksensor findet. Die Gestaltung der Einheit Mine/Drucksensor für Axiale Drücke in einem Modul vermeidet diese Schwierigkeiten. Bei der Verwendung unterschiedlicher Stiftspritzen bietet es sich zudem an, den Durchmesser der Stiftart anzupassen, deswegen befindet sich auch die Drucksensoren in diesem System. Nicht zuletzt auch deshalb um aus hygienischen Gründen für jeden Patienten ein eigenes Sensormodul zu verwenden. Um die Beschleunigungswerte für die Diagnose und Therapie einsetzen zu können ist es wichtig, dass sich der Beschleunigungssensor nahe an der Schreibfläche befindet um den direkten Zusammenhang zwischen Beschleunigung und Schriftbild ziehen zu können. Deswegen ist auch dieser Sensor in diesem ersten Modul - Stiftspitze- enthalten.

Als zweites Modul kann der Mittelteil einer erfindungsgemäßen Schreibvorrichtung gestaltet werden, in dem die Datenverarbeitungseinheit bzw. die Einrichtung zum Übertragen der erfassten Daten an eine externe Datenverarbeitungseinheit vorzusehen ist. Da im Mittelteil am meisten Platz zur Verfügung steht und um eine gute Gewichtsverteilung zu erreichen bietet es sich an auch noch die Energieversorgung im mittleren Modul der erfindungsgemäßen Schreibvorrichtung vorzusehen.

Im dritten modularen Teil der bevorzugten erfindungsgemäßen Schreibvorrichtung, dem Schlussstück, der als Schreibkappe gestaltet werden kann, kann die Einrichtung zur Erzeugung des Rückmeldesignals untergebracht werden. Sollen die Daten gespeichert werden kann hier auch beispielsweise ein Speicher mit einem USB-Anschluss vorgesehen werden, der einfach abzunehmen ist und mit jedem handelsüblichen Computer problemlos verbunden werden kann. Wenn in einer Phase der Erfassung auf die direkte Rückmeldung kein Wert gelegt wird, können auch verschiedene Stiftkappenmodule alternativ verwendet werden, die einen Datenspeicher, eine Einrichtung zur Übertragung von Daten oder eine Einrichtung zur Erzeugung des Rückmeldesignals alternativ enthalten. Neben der haptisch spürbaren oder akustischen Rückmeldung ist es bei der erfindungsgemäßen Vorrichtung in Modulbauweise auch möglich, eine optische Rückmeldung vorzusehen. Bevorzugt wird diese so gestaltet, dass dem Nutzer durch verschiedenfarbige optische Rückmeldungen beispielsweise wie bei einer Verkehrsampel durch ein grünes Signal gemeldet wird, dass er sich im richtigen Bereich befindet. Mit einer orangefarbigen Rückmeldung kann signalisiert werden, dass der Grenzwert der vorgesehenen Bereiche erreicht ist und schließlich kann eine Rückmeldung in Rot signalisieren, dass Korrekturbedarf besteht. Auf diese Weise der optischen Rückmeldung mit verschiedenfarbigen Anzeigen kann dem Nutzer während des Schreibvorgangs eine kontinuierliche Rückmeldung gegeben werden, wenn er die Schreibhaltung in unerwünschter Weise verändert. Es versteht sich, dass selbstverständlich auch beliebige andere Farbkombinationen für die optische Rückmeldung eingesetzt werden können.

Durch den modularen Aufbau ist es in dieser Variante der vorliegenden Erfindung möglich, sowohl Stiftspitze als auch Übertragungseinheit an den jeweiligen Einsatzzweck bzw. Nutzer optimal anzupassen.

Wegen des geringen Stromverbrauchs werden für die optische Rückmeldung vorzugsweise LED-Leuchteinheiten eingesetzt, die zudem auch leichter miniaturisierbar sind als konventionelle Leuchten.

Es ist ebenfalls möglich, zwei oder mehr Arten der Rückkopplung in einem Modul zu nutzen, so z.B. eine optische Rückkopplung solange sich der Nutzer im vordefinierten Bereich bewegt, die dann mit einer akustischen oder haptischen Rückmeldung kombiniert wird, sobald die Schreibhaltung sich so ändert, dass der vorgegebene Bereich verlassen wird.

Ist eine Stromversorgung nur in einem der Module vorgesehen, ist darauf zu achten, dass bei der Verbindung der Module die Stromversorgung der Sensoren bzw. Einrichtungen in allen Modulen durch entsprechende Kontakte sichergestellt ist.

In einer Variante der vorliegenden Erfindung ist es möglich, weitere Kraft- oder Beschleunigungssensoren zu verbauen.

Die Schreibstiftspitze kann im Rahmen der vorliegenden Erfindung nicht nur die reine Spitze, die die Unterlage berührt, umfassen, sondern z.B. auch eine Schreibmine (bestehend bei einem Kugelschreiber z.B. aus Kugelkopf und Tintenreservoir), einen oder mehrere der Sensoren etc.
In einer bevorzugten Variante umfasst die Schreibstiftspitze Schreibmine und zumindest einen Sensor zur Messung der axialen Kräfte.

Die erfindungsgemäße Schreibvorrichtung muss nicht zum Schreiben auf Papier ausgestaltet sein.

Ausgehend von der Variation der eingesetzten Schreibstiftspitze sind verschiedene weitere Ausgestaltungen und Anwendungen denkbar. Die Schreibstiftspitze kann in Variationen der vorliegenden Erfindung auch Elemente ausgewählt aus der Gruppe bestehend aus Kugelschreiberminen, Füllerfedern, Faserschreiberminen(spitzen), Kunststoffspitzen, Metallspitzen oder gegebenenfalls auch speziell für den jeweiligen Zweck angefertigte Spezialstücke, umfassen. Dies gilt insbesondere für den Fall, dass die erfindungsgemäß Schreibvorrichtung modular aufgebaut ist.

### Beispiele sind:

| Ausgestaltung der Schreibstiftspitze | Anwendungsbeispiele |
|---|---|
| Kugelschreibermine | Therapie, Schönschrift, akkurate Schrift |
| Füllerfeder | Therapie, Schönschrift, akkurate Schrift |
| Fasermine(nspitze) | Therapie, Schönschrift, akkurate Schrift |
| Kunststoff | LCD's, Graphiktabletts, Touchscreens |
| Metallspitze | Schnitzen, Gravieren, Chirurgenausbildung |

Die erfindungsgemäße Schreibvorrichtung kann verwendet werden
- in Verfahren zur Diagnose und Therapie neuromotorischer Störungen mittels der kontinuierlichen Erfassung biometrischer Daten bei der handgeführten Bewegung einer Schreibvorrichtung durch einen Nutzer, wobei die erfassten biometrischen Daten in einer Datenverarbeitungseinheit kontinuierlich mit gespeicherten Referenzdaten verglichen und auf eine Abweichung gegenüber vorher festgelegten Referenzdaten geprüft werden, **dadurch gekennzeichnet, dass** bei einer Abweichung von den Referenzdaten unmittelbar ein Signal generiert wird, welches unmittelbar in der Schreibvorrichtung zu einer haptisch spürbaren oder akustischen Rückmeldung an den Nutzer führt, solange die Abweichung von den Referenzdaten anhält,
- beim Malen oder Zeichnen, wodurch besonders akkurate Strichführungen ermöglicht werden,
- für Schnitz- oder Gravurarbeiten, wodurch besonders akkurate Ausarbeitungen ermöglicht werden,
- bei der Chirurgenausbildung, wodurch das genau dosierte Schneiden mit dem Skalpell geübt werden kann,
- bei Grafiktabletts oder LCD-Bildschirmen, wodurch ebenfalls besonders genaue Darstellungen ermöglicht werden.
Bevorzugt ist die Verwendung in Verfahren zur Diagnose und Therapie neuromotorischer Störungen.

Die vorliegende Erfindung wurde im wesentlichen im Hinblick auf die Kontrolle oder Überwachung der Schreibhaltung beschrieben; bei einer Variation der Schreibstiftspitze gelten aber sinngemäß die gleichen Ausführungen auch für die dann gewählte Ausführungsform, z.B. das malen o.ä. - die obigen Ausführungen sind ihrem Sinngehalt nach demnach auf Schreibgeräte im engeren Sinne beschränkt.

Die verschiedenen Ausgestaltungen der vorliegenden Erfindung, z.B. aber nicht ausschließlich diejenigen der verschiedenen abhängigen Ansprüche, können dabei in beliebiger Art und Weise miteinander kombiniert werden.

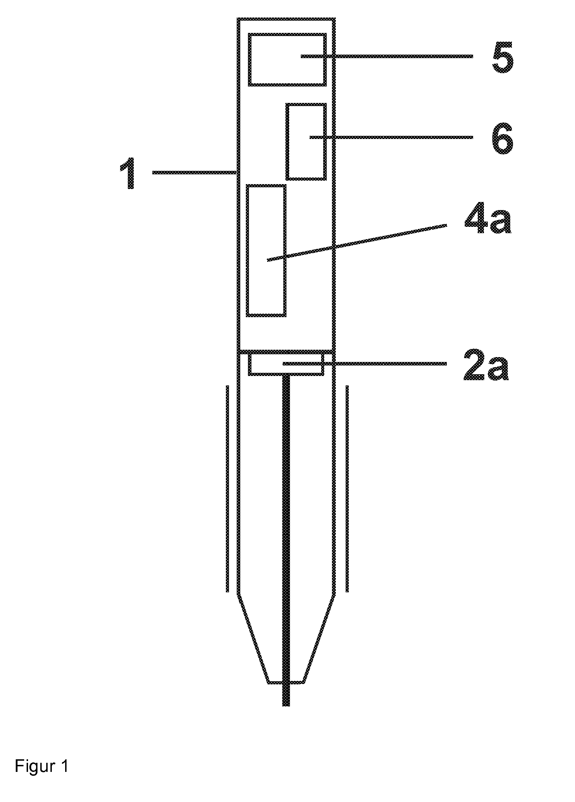

Die Erfindung wird nun unter Bezugnahme auf die Figuren 1 bis 3 näher erläutert.

### Beschreibung der Figuren:

Die Zeichnungen sind nicht unbedingt maßstabsgetreu. Aus Gründen der Klarheit und zur einfacheren Darstellung können einige Merkmale der Erfindung übertrieben groß oder in schematischer Form dargestellt sein, ebenso können demgemäß einige Details von konventionellen bzw. bekannten Elementen nicht dargestellt sein.
Bei mehreren gleichen Bauteilen/Bestandteilen in einer Zeichnung wurden aus Gründen der Übersichtlichkeit nicht alle markiert, sondern jeweils maximal drei.

### Bezugszeichenliste:

- 1: Schreibstiftgehäuse
- 2: Drucksensor
- 3: Beschleunigungssensor
- 4: Datenverarbeitungseinheit
- 5: Einrichtung zur Erzeugung und Abgabe eines haptisch spürbaren oder akustischen Signals
- 6: Energiequelle
- 7: Datenspeicher (der Übersichtlichkeit halber nicht gezeigt)
- 8: Einrichtung 8 zur Übertragung der erfassten Daten an die Datenverarbeitungseinheit 4b aufweist
- 9: erstes Modul (Schreibstiftspitze umfassend 10)
- 10: Schreibmine (Teil von 9)
- 11: zweites Modul (Mittelstück)
- 12: drittes Modul (Schlussstück)

### Figur 1:

Figur 1 zeigt eine erste erfindungsgemäße Vorrichtung mit einem Schreibstiftgehäuse 1, einem Drucksensor 2a zur Messung axialer Kräfte, einer integrierten Datenverarbeitungseinheit 4a, einer Einrichtung 5 zur Erzeugung und Abgabe eines haptisch spürbaren oder akustischen Signals und einer Energiequelle 6.

### Figur 2:

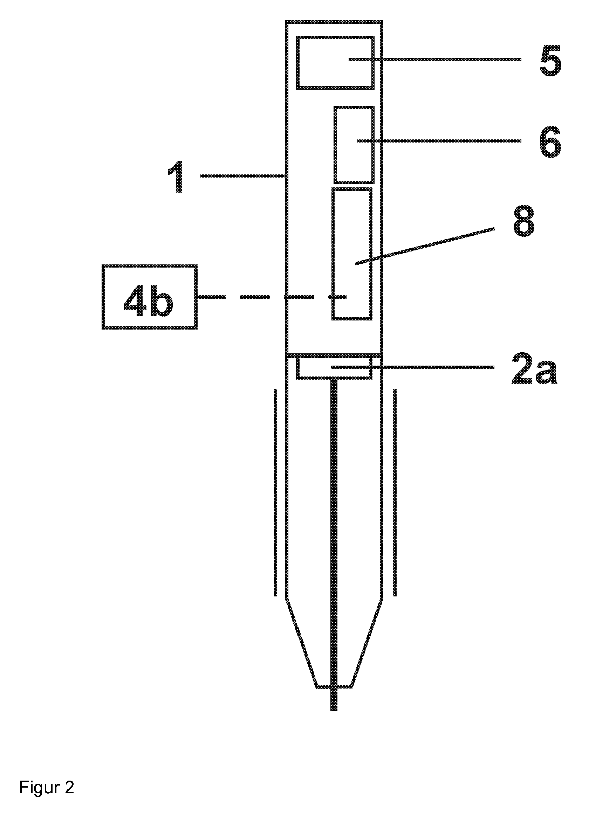

Figur 2 zeigt eine alternative Ausführungsform, bei der die Datenverarbeitungseinheit 4b außerhalb der eigentlichen Schreibvorrichtung beispielsweise in einem Laptop untergebracht ist und die Vorrichtung eine entsprechende Einrichtung 8 zur Übertragung der erfassten Daten an die Datenverarbeitungseinheit 4b aufweist.

### Figur 3:

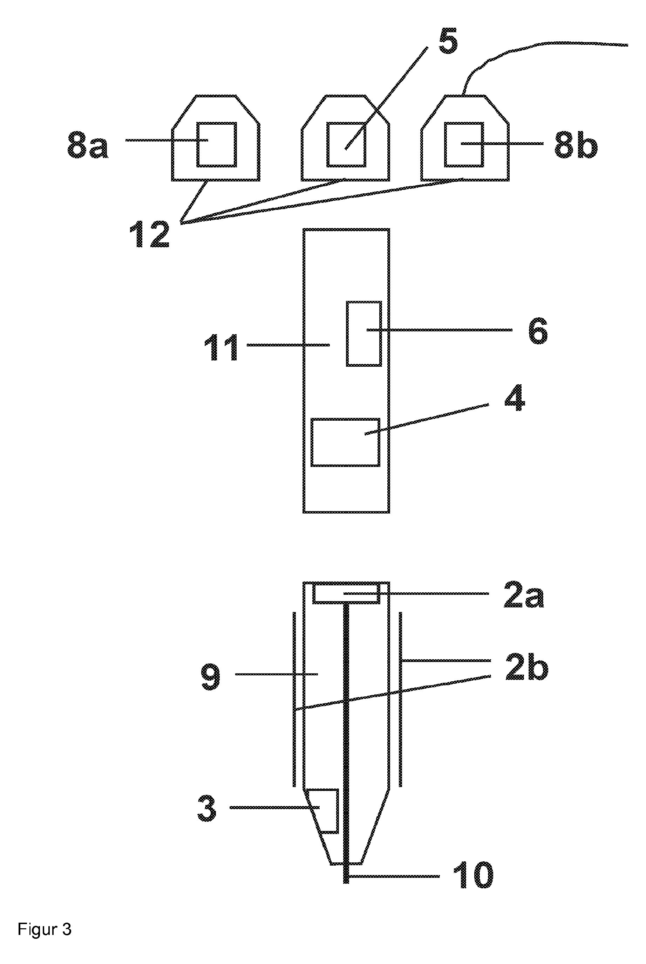

Figur 3 zeigt eine bevorzugte erfindungsgemäße Schreibvorrichtung mit drei Modulen, wovon das erste Modul 9 die Schreibstiftspitze ist, umfassend eine Schreibmine 10, einen Sensor 2a zur Messung der axialen Drücke, einen Drucksensor 2b für die Messung der radialen Drücke und einen Beschleunigungssensor 3 zur Erfassung der Beschleunigungskräfte. Das zweite Modul 11, das Mittelstück, beinhaltet eine Datenverarbeitungseinheit 4 sowie als Energieversorgung eine Batterie 6. Im dritten Modul 12, dem Schlussstück, welches als Stiftkappe gestaltet ist, ist eine Einrichtung 5 zur Erzeugung und Abgabe eines haptisch spürbaren, optischen oder akustischen Signals enthalten. Bei der gezeigten Ausführungsform ist das dritte Modul als Wechselmodul gestaltet; so kann das Modul mit der Einrichtung 5 durch ein Modul mit einer Einrichtung 8 zur Übertragung der Daten an eine externe Datenverarbeitungseinheit gestaltet sein, die entweder drahtlos (8a) oder kabelgebunden (8b) arbeitet. In den beiden äußeren Wechselmodulen 12 ist die Einrichtung 5 der Klarheit halber nicht eingezeichnet.

## Patentansprüche

1. Schreibvorrichtung umfassend
a) ein Schreibstiftgehäuse (1) zur Durchführung handgeführter Bewegungen,
a1) eine Schreibstiftspitze,
b) mindestens einen Drucksensor (2) zur Messung von axialen und mindestens einen Drucksensor zur Messung von radialen Drucken und/oder einen Beschleunigungssensor (3),
c) alternativ
c1) eine interne Datenverarbeitungseinheit (4a) zum Vergleich der mittels der Sensoren erfassten Daten mit vorher festgelegten Referenzdaten, oder
c2) eine Einrichtung (8) zur Übertragung der erfassten Daten an eine externe Datenverarbeitungseinheit (4b),
d) eine Vorrichtung (5) zur Abgabe eines haptisch spürbaren und optischen, akustischen oder optisch-akustischen Signals und
e) eine Energiequelle (6) sowie
f) ggf. einen Datenspeicher (7) zur Speicherung der erfassten Daten.

2. Schreibvorrichtung nach Anspruch 1, **dadurch gekennzeichnet, dass** die erfassten Daten über eine drahtlose Verbindung (8a) oder über eine kabelgebundene Verbindung (8b) an die Datenverarbeitungseinheit übertragen werden.

3. Schreibvorrichtung nach einem der Ansprüche 1 oder 2, **dadurch gekennzeichnet, dass** es sich bei der haptisch spürbaren Rückmeldung um eine Vibration handelt.

4. Schreibvorrichtung nach einem der Ansprüche 1 bis 3, **dadurch gekennzeichnet, dass** sie modular aufgebaut ist.

5. Schreibvorrichtung nach Anspruch 4, **dadurch gekennzeichnet, dass** das erste Modul die Schreibstiftspitze umfasst, das zweite Modul den Mittelteil der Vorrichtung und das dritte Modul das Schlussstück der Schreibvorrichtung.

6. Schreibvorrichtung nach einem der Ansprüche 1 bis 5, **dadurch gekennzeichnet, dass** neben der haptisch spürbaren oder akustischen Rückmeldung eine optische Rückmeldung erfolgt.

7. Schreibvorrichtung nach einem der Ansprüche 1 bis 6, **dadurch gekennzeichnet, dass** die erfassten Daten biometrische Daten sind und mindestens einer, bevorzugt jeder der Parameter axialer Druck, radialer Druck oder Beschleunigungskräfte erfasst werden und wobei die Beschleunigungskräfte bevorzugt in mindestens zwei Raumachsen erfasst werden.

8. Verwendung einer Schreibvorrichtung nach einem der Ansprüche 1 bis 7
- in Verfahren zur Diagnose und Therapie neuromotorischer Störungen mittels der kontinuierlichen Erfassung biometrischer Daten bei der handgeführten Bewegung einer Schreibvorrichtung durch einen Nutzer, wobei die erfassten biometrischen Daten in einer Datenverarbeitungseinheit kontinuierlich mit gespeicherten Referenzdaten verglichen und auf eine Abweichung gegenüber vorher festgelegten Referenzdaten geprüft werden, **dadurch gekennzeichnet, dass** bei einer Abweichung von den Referenzdaten unmittelbar ein Signal generiert wird, welches unmittelbar in der Schreibvorrichtung zu einer haptisch spürbaren oder akustischen Rückmeldung an den Nutzer führt, solange die Abweichung von den Referenzdaten anhält,
- beim Malen oder Zeichnen,
- für Schnitz- oder Gravurarbeiten,
- bei Grafiktabletts oder LCD-Bildschirmen
- bei der Chirurgenausbildung.

9. Verwendung einer Schreibvorrichtung nach einem der Ansprüche 1 bis 7 zur Kontrolle der Schreibhaltung oder in einem Verfahren zur Diagnose und/oder Therapie neuromotorischer Störungen, insbesondere graphomotorischer Störungen.
